# FASCICULE DE BREVET EUROPEEN

(11) **EP 0 919 199 B1**
(45) Date de publication et mention de la délivrance du brevet: **26.01.2005**
(21) Numéro de dépôt: 98420214.3
(22) Date de dépôt: 26.11.1998
(51) Int. Cl.: A61B 17/70

(54) **Dispositif de liaison intervertébrale à débattement axial et angulaire**
Zwischenwirbel-Verbindungsvorrichtung mit Axialspiel und Winkelspiel
Intervertebral connecting device with axial and angular play

(30) Priorité: 26.11.1997 FR 9714831
(43) Date de publication de la demande: 02.06.1999
(73) Titulaire: SCIENT'X S.A.R.L., 75007 Paris (FR)
(72) Inventeur: Alby, Albert, 75116 Paris (FR)
(74) Mandataire: Thibault, Jean-Marc

(56) Documents cités:
- EP-A- 0 677 277
- FR-A- 2 697 428
- FR-A- 2 697 993
- FR-A- 2 730 918

## Description

La présente invention concerne un dispositif de liaison intervertébrale destiné, notamment, au traitement de défauts ou d'états pathologiques de la colonne vertébrale ou de vertèbres.

L'objet de l'invention concerne, plus particulièrement, un dispositif de stabilisation intervertébrale destiné au maintien en position relative convenable, d'au moins deux vertèbres, en vue de corriger chez un patient, par exemple, un tassement des vertèbres, une scoliose, une lordose, une cyphose ou une instabilité intervertébrale.

L'objet de l'invention trouve une autre application dans le domaine des liaisons articulaires ou osseuses effectuées de manière tant interne, qu'externe.

Dans le domaine préféré d'application de l'ostéosynthèse du rachis, il existe de nombreux systèmes composés d'éléments de liaison qui s'appliquent à la face postérieure des vertèbres et sont fixés au moyen de vis implantées dans les pédicules vertébraux.

Ce type de montage permet de solidariser plusieurs vertèbres entre elles, de manière à obtenir une arthrodèse ou une fusion osseuse.

Dans le domaine technique ci-dessus, il est connu une première catégorie de dispositifs comportant au moins deux plaques de liaison, pourvues de lumières et disposées longitudinalement de part et d'autre des apophyses épineuses, et au moins quatre vis de fixation disposées par paire sur les pédicules de part et d'autre de deux vertèbres successives. Les extrémités filetées libres desdites vis traversent les lumières desdites plaques dont la fixation définitive après réglage est assurée par des écrous vissés sur les extrémités filetées.

L'avantage principal de telles plaques de liaison réside dans leur rigidité. Néanmoins, il s'est souvent avéré qu'une telle rigidité était parfois également un inconvénient, car elle ne permet pas au chirurgien d'adapter les plaques à la morphologie des vertèbres en fonction du patient.

Il est connu, par ailleurs, une deuxième catégorie de dispositifs de liaison intervertébrale constitués à partir d'une tige de section circulaire fixée sur des vis d'ancrage osseux. Un avantage des tiges de liaison réside dans le fait que leur section circulaire d'un diamètre compris entre 4 et 7 mm, permet au chirurgien de les conformer à la demande par l'intermédiaire de pinces appropriées.

Toutefois, il apparaît que la mise en oeuvre d'un dispositif de stabilisation intervertébrale de type à plaque ou à tige, constitue un système rigide, ce qui conduit à un report des contraintes mécaniques sur les articulations intervertébrales adjacentes à celle stabilisée.

Pour remédier à ce problème, le document **EP 0 516 567** a proposé un dispositif de stabilisation intervertébrale réalisé sous la forme d'un amortisseur propre à résister élastiquement à un allongement et à une compression axiale. Un tel dispositif de stabilisation est capable d'amortir le mouvement en compression, comme celui en extension, autorisant ainsi un travail asymétrique des facettes des vertèbres. Toutefois, il s'avère qu'un tel dispositif de stabilisation intervertébrale consistant à amortir les mouvements en compression-extension, ne donne pas entière satisfaction.

Le document FR 2 697 428 a proposé un dispositif de liaison intervertébrale comportant un élément amortisseur constitué d'une cage et d'un axe destinés à être raccordés à des éléments d'ancrage osseux. L'axe est monté à l'intérieur d'un logement de la cage selon une articulation autorisant relativement entre-eux, un pivotement multidirectionnel. L'axe est équipé de deux organes déformables élastiquement fonctionnant de manière antagoniste selon l'application d'un effort de traction ou de compression.

En effet, il est apparu nécessaire de disposer d'un dispositif de stabilisation intervertébrale adapté pour amortir les mouvements de compression et de traction axiales, mais également les mouvements en flexion-extension dans le plan antéro-postérieur et en flexion latérale.

Cependant, il est recherché d'adapter le niveau de réduction de mobilité des vertèbres à stabiliser en fonction des cas et cela en autorisant une flexion latérale déterminée et une certaine compression et traction axiales, cette faible mobilité étant prédéterminée pour absorber les contraintes majeures, tout en autorisant des micro-mouvements réputés favorables à la fusion osseuse

L'objet de l'invention vise donc à satisfaire ce besoin en proposant un dispositif de liaison intervertébrale conçu pour amortir les mouvements de compression et de traction axiales, ainsi que les mouvements en flexion-extension dans le plan antéro-postérieur et en flexion latérale.

Un autre objectif de l'invention est de proposer un dispositif de stabilisation intervertébrale adapté pour autoriser les micro-mouvements pour assurer une fusion osseuse.

Un autre objectif de l'invention est de proposer un dispositif de liaison intervertébrale à débattement axial et angulaire présentant une grande fiabilité de fonctionnement.

Pour atteindre les divers objectifs ci-dessus, l'objet de l'invention concerne un dispositif de liaison intervertébrale conforme à la revendication 1.

Diverses autres caractéristiques ressortent de la description faite ci-dessous en référence aux dessins annexés qui montrent, à titre d'exemples non limitatifs, des formes de réalisation et de mise en oeuvre de l'objet de l'invention.
La **fig. 1** est une vue en coupe longitudinale d'un élément de liaison intervertébrale comprenant un amortisseur intercalaire conforme à l'invention.
La **fig. 2** est une vue de dessus selon la **fig. 1**.
La **fig. 3** représente un élément de liaison équipé d'un amortisseur intercalaire selon une variante de réalisation.
La **fig. 4** est une vue en coupe illustrant une autre variante de réalisation d'un élément de liaison équipé d'un amortisseur intercalaire conforme à l'invention.
La **fig. 5** est une vue de dessus de l'élément de liaison illustré à la **fig. 4**.

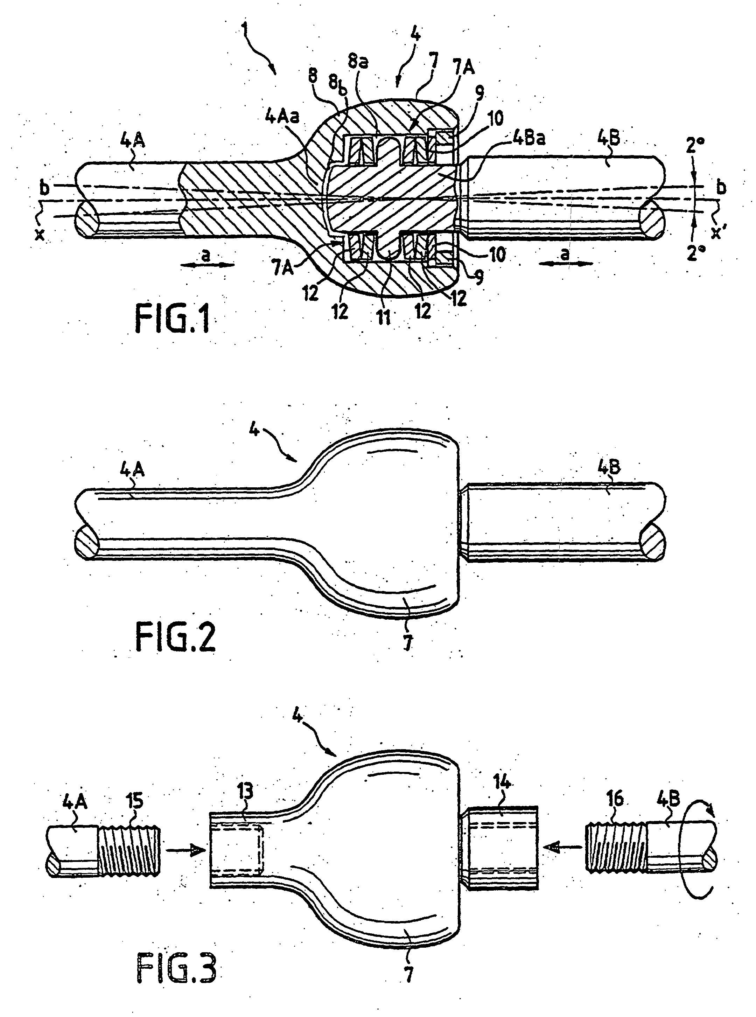

L'objet de l'invention concerne un dispositif **1** destiné à assurer une liaison entre au moins deux vertèbres en étant destiné à être raccordé à des implants ou à des éléments d'ancrage osseux, tels que des vis pédiculaires. Dans l'exemple illustré aux **fig. 1** à **3**, les implants non représentés sont reliés entre-eux par une tige de liaison **4** constituée par au moins deux segments rigides **4A, 4B** raccordés entre-eux par l'intermédiaire d'un élément amortisseur **7** intercalé entre leur extrémité libre située en vis-à-vis, de manière à opposer une résistance élastique entre les segments **4A** et **4B** selon une amplitude contrôlée tant en compression et traction axiales **a**, qu'en flexion angulaire **b**.

Bien entendu, une même tige de liaison **4** peut comporter plusieurs amortisseurs **7** disposés entre les vertèbres. Egalement, la tige de liaison **4** peut être avantageusement coupée suivant une longueur et cintrée suivant un rayon choisis.

Comme cela apparaît plus précisément à la **fig. 1**, l'élément amortisseur **7** est constitué par deux organes déformables élastiquement **7A**, disposés autour de l'extrémité libre d'un axe **4Ba** prolongeant l'un des segments **4B** constitutif de la tige **4**. L'axe **4Ba** est engagé à l'intérieur d'un logement **8a** présenté par un manchon borgne ou une cage **8** réalisé à l'extrémité libre **4Aa** de l'autre segment de liaison **4A**. Selon l'exemple de réalisation représenté à la **fig. 1**, l'élément amortisseur **7** comprend un piston rigide **11** aménagé sur l'axe **4Ba** pour constituer une articulation autorisant entre la cage **8** et l'axe **4Ba**, un pivotement multidirectionnel relativement entre l'axe **4Ba** et la cage **8**, selon au moins des axes contenus dans un plan perpendiculaire à l'axe longitudinal **x-x'** de l'élément amortisseur **7**, considéré lorsque l'axe et la cage sont alignés.

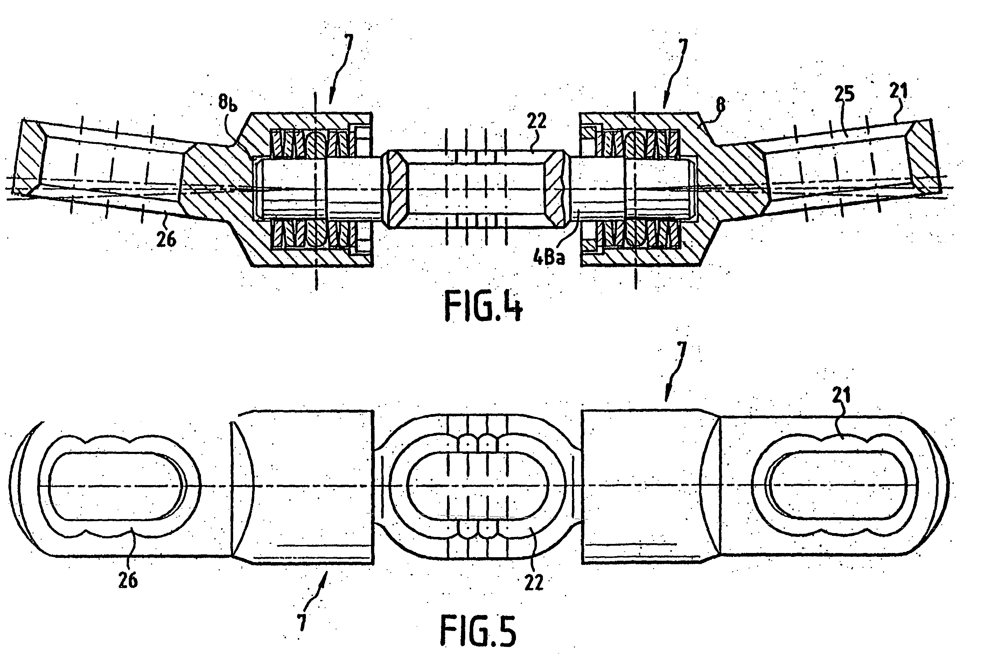

Selon une caractéristique préférée de réalisation, l'articulation **11** formée est du type à rotule permettant également, autour de l'axe **x-x'**, une rotation relative de la cage par rapport à l'axe. L'articulation **11** est constituée par un collet s'étendant radialement à partir de l'axe **4Ba** et présentant une surface d'extrémité avec un profil arrondi, destinée à être en contact avec la surface interne du logement **8a** de la cage **8**. Dans l'exemple illustré à la **fig. 1**, le collet **11** est réalisé à partir de l'axe **4Ba**, tandis que dans l'exemple illustré à la **fig. 4**, le collet **11** est constitué à partir d'une bague rapportée et montée fixement sur l'axe **4Ba**.

Le collet **11** est aménagé sur l'axe **4Ba**, de manière à recevoir en appui, de part et d'autre de ses faces latérales, un jeu de deux rondelles **12** formant des cuvettes tronconiques opposées, de diamètre identique, disposées face à face et empilées deux à deux sur l'axe **4Ba**. L'ensemble des rondelles **12** et l'articulation **11** occupent au moins en partie, le logement **8a** de section circulaire, dont le fond sert de butée en compression pour l'un des organes élastiquement déformables **7A**. Il est à noter que les rondelles **12** connues sous le nom de rondelles Belleville, peuvent être remplacées par des bagues en élastomère.

Selon une autre caractéristique de l'invention, la fermeture du logement **8a** de la cage **8** est effectuée par l'intermédiaire d'une première rondelle **9** solidaire de la cage **8** et sur une face interne de laquelle prend appui une seconde rondelle **10**. Les organes déformables **7A** sont disposés librement sur l'axe **4Ba** entre la rondelle **10** et le fond du logement **8a**. Par exemple, la première rondelle **9**, qui sert de butée axiale, est réalisée sous la forme d'une bague filetée, vissée sur un taraudage réalisé à l'intérieur du logement à partir de son extrémité externe, permettant de régler la position en extension de l'amortisseur. Il est à noter que la seconde rondelle **10**, constitue une surface d'appui pour un organe élastiquement déformable **7A**. Cette seconde rondelle **10** sert de butée en traction axiale de l'amortisseur. Cette seconde rondelle **10** permet ainsi d'exercer l'effort de compression sur l'organe déformable sans entraîner sa détérioration. De plus, selon une caractéristique avantageuse, la seconde rondelle **10** peut être réalisée en un matériau identique à celui constitutif de l'organe déformable élastiquement, de manière à permettre de contrôler la friction apparaissant entre la seconde rondelle **10** et l'organe élastiquement déformable **7A**.

Les organes élastiquement déformables **7A** sont maintenus avec un jeu axial permettant, par leur déformation élastique, les mouvements de compression et de traction axiales **a** relatif entre l'axe **4Ba** et la cage **8**. Par exemple, il peut être obtenu une compression ou une traction axiale d'une valeur **a** = ± 0,8 mm. De plus, les organes élastiquement déformables **7A** sont montés de manière à autoriser un pivotement relatif multidirectionnel entre l'axe **4Ba** et la cage **8**. A cet égard, les rondelles sont montées à l'intérieur du logement **8a** avec un jeu par rapport à la paroi interne du logement.

Selon une caractéristique avantageuse de l'invention, l'élément amortisseur **7** comporte des moyens de butée angulaire permettant de limiter le pivotement relatif multidirectionnel à une valeur déterminée de l'ordre de 4 degrés d'amplitude. Ainsi, tel que cela apparaît plus précisément à la **fig. 1**, le déplacement **b** de l'axe **4Ba** de la cage **8** par rapport à la position normale d'alignement est de ± 2 degrés. Dans l'exemple illustré, les moyens de butée angulaire sont constitués par le logement **8a** sur lequel vient en butée l'axe **4Ba** qui présente avec le logement **8a**, un jeu radial prédéterminé pour autoriser le pivotement relatif selon la valeur prédéterminée **b**. Ainsi, l'axe **4Ba** possède un jeu radial, d'une part, entre son collet **11** et le logement **8a** et, d'autre part, entre son extrémité libre et un évidement borgne **8b** prolongeant le logement **8a**. La limitation du pivotement relatif entre la cage **8** et l'axe **4Ba** est donc assurée par la mise en oeuvre de deux butées angulaires définies par la coopération entre, d'une part, le collet **11** et le logement **8a** et, d'autre part, l'extrémité libre de l'axe et l'évidement borgne **8b**. Il est à noter que les deux butées ainsi constituées s'établissent en opposition de part et d'autre de l'axe **x-x'**. Il peut ainsi être obtenu une flexion limitée entre la cage et l'arbre dans toutes les directions de décalage angulaire.

Selon une variante de réalisation représentée à la **fig. 3**, l'élément amortisseur **7** est un élément indépendant comportant des zones d'extrémités axiales **13** et **14** destinées au raccordement par filetage, d'extrémités correspondantes **15** et **16** respectivement des segments **4A** et **4B** pour une tige de liaison **4**. Bien entendu, les segments peuvent être de longueur variable et choisis dans une gamme en fonction du cas traité. Par exemple, les extrémités des segments **4A** et **4B** de la tige de liaison **4** comportent des filetages destinés à être vissés dans des taraudages aménagés sur les parties d'extrémités axiales **13** et **14** de l'élément amortisseur **7**. Dans le cas où il est prévu de réaliser une tige **4** comportant plusieurs amortisseurs, il sera utilisé des segments de longueur prédéterminée et filetés à leurs deux extrémités.

L'élément amortisseur **7**, qui vient d'être décrit ci-dessus, peut s'adapter sur un élément de liaison différent du type tige. A cet effet, les **fig. 4** et **5** illustrent un autre exemple de réalisation pour lequel un élément amortisseur **7** est destiné à être associé à des plaques de section rectangulaire. Dans l'exemple illustré, le dispositif de liaison intervertébrale **1** comprend un élément amortisseur **7** dont la cage **8** est prolongée par une première plaque **21**, tandis que l'axe **4Ba** est prolongé par une deuxième plaque **22** de section générale rectangulaire. Les plaques **21** et **22** comportent classiquement des passages axiaux **25** pour des implants osseux.

Dans l'exemple illustré à la **fig. 4**, le dispositif de liaison intervertébrale **1** comprend un deuxième élément amortisseur **7** dont l'axe **4Ba** est relié à la seconde plaque de liaison **22**, tandis que la cage **8** est prolongée par une troisième plaque **26**.

## Revendications

1. Dispositif de liaison intervertébrale comportant au moins un élément amortisseur (**7**) constitué d'une cage (**8**) et d'un axe (**4Ba**) destinés à être raccordés à des éléments d'ancrage osseux, l'axe (**4Ba**) étant monté à l'intérieur d'un logement (**8a**) de la cage **(8)** selon une articulation (**11**) autorisant, relativement entre-eux, un pivotement multidirectionnel selon au moins des axes contenus dans un plan perpendiculaire à l'axe, et en étant équipé de deux organes déformables (7A) élastiquement fonctionnant de manière antagoniste selon l'application d'un effort de traction ou de compression,
**caractérisé en ce que** l'élément amortisseur (**7**) comporte :
- une première rondelle (**9**) montée solidaire de la cage (**8**), et sur laquelle prend appui une seconde rondelle (**10**) disposée sur l'axe (**4Ba**) et qui sert de butée en traction axiale (**10**) de l'amortisseur (**7**) et permet d'exercer l'effort de compression sur l'un des organes déformables (**7A**) sans entraîner sa détérioration,
- et des moyens de butée angulaire entre la cage (**8**) et l'axe (**4Ba**) permettant de limiter le pivotement relatif multidirectionnel à une valeur de l'ordre de 4 degrés d'amplitude.

2. Dispositif selon la revendication 1, **caractérisé en ce que** les moyens de butée angulaire sont constitués par le logement (**8a**) sur lequel vient en butée l'axe (**4Ba**) qui présente avec le logement (**8a**), un jeu radial prédéterminé pour autoriser le pivotement relatif selon la valeur déterminée.

3. Dispositif selon la revendication 2, **caractérisé en ce que** l'axe (**4Ba**) possède un jeu radial, d'une part, entre son articulation (**11**) et le logement et, d'autre part, son extrémité libre et un évidement borgne (**8b**) prolongeant le logement (**8a**), de manière à autoriser le pivotement et à constituer deux butées angulaires.

4. Dispositif selon la revendication 1, **caractérisé en ce que** l'élément amortisseur (**7**) est constitué par deux organes déformables élastiquement (**7A**) disposés autour de l'extrémité libre (**4Ba**) de l'axe (**4**) et de part et d'autre de l'articulation, les organes déformables élastiquement étant montés à l'intérieur de la cage en étant retenus avec un jeu axial et angulaire prédéterminé, les mouvements relatifs de la cage par rapport à l'axe étant obtenus par la déformation élastique des organes déformables (**7A**) et par le jeu prédéterminé.

5. Dispositif selon l'une des revendications 1 ou 4, **caractérisé en ce que** l'élément amortisseur (**7**) comprend une articulation constituée par un collet porté par l'axe et recevant en appui, de part et d'autre de ses faces latérales, un jeu de deux rondelles **(12)** formant des cuvettes tronconiques opposées de diamètres identiques disposées face à face et empilées deux à deux sur l'extrémité **(4Ba)** de l'axe de chaque côté du piston **(11)**, pour que l'ensemble des rondelles **(12)** et l'articulation **(11)** occupent l'espace annulaire défini entre l'extrémité de l'axe **(4Ba)** et la paroi interne de la cage.

6. Dispositif selon l'une des revendications précédentes, **caractérisé en ce que** l'élément amortisseur **(7)** est un élément indépendant comportant des parties de raccordement d'extrémités axiales **(13)** et **(14)** destinées au raccordement d'extrémités correspondantes **(15)** et **(16)** pour des tiges de liaison **(4)** ou de plaque de liaison.

7. Dispositif selon la revendication 6, **caractérisé en ce que** les parties de raccordement **(13, 14)** et **(15, 16)**, respectivement de l'élément amortisseur **(7)** et des extrémités des tiges **(4)** ou des plaques de liaison **(21, 22, 26)** sont constituées par des parties filetées.

8. Dispositif selon l'une des revendications 1 à 5, **caractérisé en ce que** l'élément amortisseur **(7)** comporte un axe **(4Ba)** prolongé par une tige ou une plaque de liaison et une cage prolongée par une tige ou une plaque de liaison.

9. Dispositif selon la revendication 1, **caractérisé en ce que** la butée axiale **(9)** est réalisée sous la forme d'une bague filetée permettant de régler la position en extension de l'amortisseur.

## Claims

1. An intervertebral link device including at least a damping component (7) consisting of a cage (8) and of an axis (4Ba) intended to be connected to bone anchoring members, the axis (4Ba) being mounted inside a housing (8a) of the cage (8) along a joint (11) allowing, relatively to each other, multidirectional pivoting along at least axes contained in a plane perpendicular to the axes, and being equipped therein with two elastically deformable units (7A) operating antagonistically according to the application of a tensile or compressive stress,
**characterized in that**, the damping component (7) includes:
- a first mounted washer (9) firmly attached to the cage (8), and on which is supported a second washer (10) positioned on the axis (4Ba) and which is used as a stop under axial tensile loading (10) of the damper (7) and allows the compression stress to be exerted on one of the deformable members (7A) without causing its deterioration,
- and angular stop means between the cage (8) and the axis (4Ba) allowing the multidirectional relative pivoting to be limited to a value of the order of 4 degrees in amplitude.

2. The device according to claim 1, **characterized in that** the angular stop means are formed by the housing (8a) onto which abuts the axis (4Ba) which has with the housing (8a), a predetermined radial play for allowing relative pivoting according to the determined value.

3. The device according to claim 2, **characterized in that**, the axis (4Ba) has a radial play between its joint (11) and the housing on the one hand, and on the other hand, between its free end and a blind recess (8b) extending the housing (8a), so as to allow the pivoting and form two angular stops.

4. The device according to claim 1, **characterized in that** the damping component (7) is formed by two elastically deformable members (7A) positioned around the free end (4Ba) of the axis (4) and on either side of the joint, the elastically deformable members being mounted inside the cage, and being retained there with a predetermined axial and angular play, the relative movements of the cage with respect to the axis being obtained by the elastic deformation of the deformable members (7A) and by the predetermined play.

5. The device according any of claims 1 or 4, **characterized in that** the damping component (7) comprises a joint formed by a collar borne by the axis and receiving in support, on either side of its side faces, a set of two washers (12) forming opposite tapered cups of identical diameters positioned face-to-face and stacked two-by-two on the end (4Ba) of the axis of each side of the piston (11), so that the whole of the washers (12) and of the joint (11) occupy the annular space defined between the end of axis (4Ba) and the internal wall of the cage.

6. The device according any of the preceding claims, **characterized in that** the damping component (7) is an independent component including portions for connecting axial ends (13) and (14) intended for connecting matching ends (15) and (16) for link rods (4) or of link plate.

7. The device according to claim 6, **characterized in that** the portions (13, 14) and (15, 16) for connecting the damping component (7) and the ends of the rod (4) or the link plates (21, 22, 26), respectively, are formed by threaded portions.

8. The device according to any of claims 1 to 5, **characterized in that** the damping component (7) includes an axis (4Ba) extended by a rod or a link plate and a cage extended by a rod or a link plate.

9. The device according to claim 1, **characterized in that** the axial stop (9) is made as a threaded ring allowing the extended position of the damper to be adjusted.

## Patentansprüche

1. Zwischenwirbel-Verbindungsvorrichtung, umfassend wenigstens ein Dämpfungselement (7), das aus einem Käfig (8) und einer Achse (4Ba) ausgebildet wird, die dazu bestimmt sind, mit knöchernen Verankerungselementen verbunden zu werden, wobei die Achse (4Ba) im Inneren einer Aufnahme (8a) des Käfigs (8) über eine Gelenkverbindung (11) befestigt ist, die relativ zwischen ihnen eine multidirektiohale Schwenkbewegung über wenigstens Achsen zu gestattet, die in einer zu der Achse senkrechten Ebene enthalten sind, und die mit zwei verformbaren Organen (7A) ausgestattet ist, die elastisch entgegenwirkend arbeiten je nach Anwendung einer Zug- oder Druckbelastungskraft,
**dadurch gekennzeichnet, daß** das Dämpfungselement (7) folgendes umfaßt:
- eine erste Unterlegscheibe (9), die mit dem Käfig (8) fest verbunden ist, und auf der eine zweite Unterlegscheibe (10) zur Auflage kommt, die auf der Achse (4Ba) angeordnet ist, und die als Anschlag (10) bei axialer Zugbelastung des Dämpfungselements (7) dient und es ermöglicht, die Druckbelastungskraft auf eines der verformbaren Organe (7A) auszuüben, ohne dessen Beschädigung mit sich zu bringen,
- und Winkel-Anschlagmittel zwischen dem Käfig (8) und der Achse (4Ba), die es gestattet, die relative multidirektioinale Schwenkbewegung auf einen Wert in der Größenordnung von 4 Winkelgrad der Schwenkweite zu begrenzen.

2. Vorrichtung nach Anspruch 1, **dadurch gekennzeichnet, daß** die Winkel-Anschlagmittel durch die Aufnahme (8a) ausgebildet werden, auf der die Achse (4Ba) zum Anschlag kommt, die mit der Aufnahme (8a) ein vorgegebenes Radialspiel aufweist, um die relative Schwenkbewegung gemäß dem vorgegebenen Wert zu gestatten.

3. Vorrichtung nach Anspruch 2, **dadurch gekennzeichnet, daß** die Achse (4Ba) ein Radialspiel aufweist einerseits zwischen ihrer Gelenkverbindung (11) und der Aufnahme und andererseits ihrem freien Ende und einer blinden Aussparung (8b), welche die Aufnahme (8a) so verlängert, daß die Schwenkbewegung gestattet wird und um zwei Winkelanschläge ausbilden.

4. Vorrichtung nach Anspruch 1, **dadurch gekennzeichnet, daß** das Dämpfungselement (7) aus zwei elastisch verformbaren Organen (7A) ausgebildet wird, die um das freie Ende (4Ba) der Achse (4) und zu beiden Seiten der Gelenkverbindung angeordnet sind, wobei die elastisch verformbaren Organe im Inneren des Käfigs befestigt sind, wobei sie mit einem vorgegebenen Axial- und Winkelspiel festgehalten werden, wobei die relativen Bewegungen des Käfigs relativ zur die Achse durch die elastische Verformung der verformbaren Organe (7A) und durch das vorgegebene Spiel erhalten werden.

5. Vorrichtung nach einem der Ansprüche 1 oder 4, **dadurch gekennzeichnet, daß** das Dämpfungselement (7) eine Gelenkverbindung aufweist, die durch einen Kragen ausgebildet wird, der von der Achse getragen wird und in Auflage von beiden seitlichen Flächen ein Spiel von zwei Unterlegscheiben (12) aufnimmt, die gegenüberliegende kegelförmige Schälchen mit identischen Durchmessern ausbilden, die gegenüberliegend angeordnet sind und jeweils zu zweit auf dem Ende (4Ba) der Achse jeder Seite des Kolbens (11) aufeinander geschichtet sind, damit die Einheit aus den Unterlegscheiben (12) und der Gelenkverbindung (11) den ringförmigen Raum einnimmt, der zwischen dem Ende der Achse (4Ba) und der Innenwand des Käfigs definiert wird.

6. Vorrichtung nach einem der vorhergehenden Ansprüche, **dadurch gekennzeichnet, daß** das Dämpfungselement (7) ein unabhängiges Element ist, das Teile (13) und (14) zur Verbindung von axialen Enden umfaßt, die für die Verbindung von entsprechenden Enden (15) und (16) für Verbindungsstäbe (4) oder Verbindungsplatten bestimmt sind.

7. Vorrichtung nach Anspruch 6, **dadurch gekennzeichnet, daß** die Verbindungsteile (13, 14) und (15, 16) jeweils des Dämpfungselements (7) und der Enden der Verbindungsstäbe (4) oder -platten (21, 22, 26) aus mit Gewinden versehenen Teilen ausgebildet werden.

8. Vorrichtung nach einem der Ansprüche 1 bis 5, **dadurch gekennzeichnet, daß** das Dämpfungselement (7) eine Achse (4Ba) umfaßt, die durch einen Verbindungsstab oder eine Verbindungsplatte verlängert wird, und einen Käfig, der durch einen Verbindungsstab oder eine Verbindungsplatte verlängert wird.

9. Vorrichtung nach Anspruch 1, **dadurch gekennzeichnet, daß** der axiale Anschlag (9) in Form eines mit einem Gewinde versehenen Rings ausgeführt ist, der das Regulieren der Position bei Ausdehnung des Dämpfungselements gestattet.
